# EUROPEAN PATENT APPLICATION

(11) **EP 4 197 588 A1**
(43) Date of publication of application: **21.06.2023**
(21) Application number: 21944168.0
(22) Date of filing: 03.06.2021
(51) Int. Cl.: A61N 1/36

(54) **EXERCISE THERAPY DEVICE**

(71) Applicant: Mitsubishi Electric Engineering Company, Limited, Chiyoda-ku Tokyo 102-0073 (JP)
(72) Inventor: MIZUKURA, Isao, Tokyo 102-0073 (JP); KIMURA, Yuichi, Tokyo 102-0073 (JP); OGISO, Kazuyuki, Tokyo 150-8366 (JP)
(74) Representative: Hoffmann Eitle
(86) International application number: PCT/JP2021/021244
(87) International publication number: WO 2022/254672

(57) **Abstract**

Provided is an exercise therapy apparatus with which a configuration of the exercise therapy apparatus can be simplified. The exercise therapy apparatus includes: an exercise device configured to cause an exercise target site of a body to mechanically exercise; and an electrical stimulation device configured to apply electrical stimulation to a pair of antagonistic muscles included in the exercise target site regardless of a form of the exercise target site. The exercise device is configured to cause the exercise target site to mechanically exercise while the electrical stimulation device applies electrical stimulation to each of the pair of antagonistic muscles at the same time.

## Description

### Technical Field

This disclosure relates to an exercise therapy apparatus for applying electrical stimulation to an exercise target site of a body.

### Background Art

Hitherto, there has been known an exercise therapy apparatus including pedals, knee angle sensors, electrodes, and a control device. On each of the pedals, a distal end part of a leg of a user who is to perform a pedaling exercise is put. The pedals are rotated by the legs of the user. The knee angle sensors and the electrodes are mounted to the legs of the user. The knee angle sensors measure angles of the knees in the legs of the user. Measurement results obtained by the knee angle sensors are input to the control device. The control device controls supply of currents to the electrodes based on information on the angles of the knees measured by the knee angle sensors. Through supply of currents to the electrodes, electrical stimulation is applied to the legs of the user. When the leg of the user is to stretch, electrical stimulation is applied to the quadriceps femoris of the leg of the user, and when the leg of the user is to bend, electrical stimulation is applied to the hamstring of the leg of the user. In this manner, the strength of the legs of the user performing the pedaling exercise is increased. As a result, the muscles of the legs of the user are developed (see, for example, Patent Literature 1).

### Citation List

### Patent Literature

[PTL 1] JP 2018-33620 A

### Summary of Invention

### Technical Problem

However, in the related-art exercise therapy apparatus, the knee angle sensors or the electrodes are required to measure the knee angles or the muscle currents in the legs of the user, and the control device is required to control the supply of currents to the electrodes based on the measured knee angles or muscle currents. As a result, there has been a problem in that the exercise therapy apparatus has a complicated configuration and has a large number of items to be adjusted.

This disclosure has been made to solve the above-mentioned problem, and has an object to provide an exercise therapy apparatus with which a configuration of the exercise therapy apparatus can be simplified.

### Solution to Problem

According to one embodiment of this disclosure, there is provided an exercise therapy apparatus including: an exercise device configured to cause an exercise target site of a body to mechanically exercise; and an electrical stimulation device configured to apply electrical stimulation to a pair of antagonistic muscles included in the exercise target site regardless of a form of the exercise target site, wherein the exercise device is configured to cause the exercise target site to mechanically exercise while the electrical stimulation device applies electrical stimulation to each of the pair of antagonistic muscles at the same time.

### Advantageous Effects of Invention

According to the exercise therapy apparatus of this disclosure, the configuration of the exercise therapy apparatus can be simplified.

### Brief Description of Drawings

FIG. 1 is a configuration diagram for illustrating an exercise therapy apparatus according to a first embodiment of this disclosure.
FIG. 2 is a block diagram for illustrating a configuration of an exercise device of FIG. 1.
FIG. 3 is a block diagram for illustrating a configuration of an electrical stimulation device of FIG. 1.
FIG. 4 is a chart for showing a waveform of a current to be supplied to an electrode of FIG. 1.
FIG. 5 is a table for showing a ratio of muscle strengths of a user before and after getting an exercise therapy using the exercise therapy apparatus according to the first embodiment.
FIG. 6 is a bar graph for showing the ratio of the muscle strengths of the user of FIG. 5.
FIG. 7 is a table for showing a ratio of muscle strengths of a user before and after getting an exercise therapy using the exercise therapy apparatus according to the first embodiment.
FIG. 8 is a bar graph for showing the ratio of the muscle strengths of the user of FIG. 7.

### Description of Embodiments

### First Embodiment

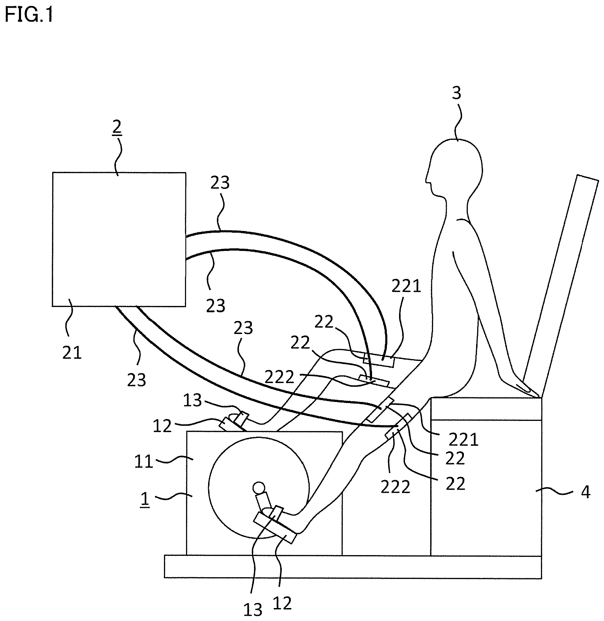

FIG. 1 is a configuration diagram for illustrating an exercise therapy apparatus according to a first embodiment of this disclosure. The exercise therapy apparatus according to the first embodiment includes an exercise device 1, an electrical stimulation device 2, and a seat 4 on which a user 3 of the exercise therapy apparatus is to sit. The exercise therapy apparatus may further include a handle (not shown) to be grasped by the user 3.

The exercise device 1 causes an exercise target site of a body of the user 3 to mechanically exercise. In the first embodiment, description is given of an example in which the exercise target site of the body of the user 3 is a leg of the user 3. The exercise target site of the body of the user 3 is not limited to the leg of the user 3, and may be, for example, an arm of the user 3.

The electrical stimulation device 2 applies electrical stimulation to a pair of antagonistic muscles included in the exercise target site of the body of the user 3. In the first embodiment, description is given of an example in which one of the pair of antagonistic muscles to which electrical stimulation is to be applied by the electrical stimulation device 2 is the quadriceps femoris and the other thereof is the hamstring. The pair of antagonistic muscles to which electrical stimulation is to be applied by the electrical stimulation device 2 are not limited to the quadriceps femoris and the hamstring, and may be another type of pair of antagonistic muscles.

The exercise device 1 includes an exercise device main body 11, a pair of pedals 12, and a pair of fixing portions 13. The pair of pedals 12 are rotatably provided to the exercise device main body 11. The pair of fixing portions 13 are provided one by one to the pair of respective pedals 12. The fixing portion 13 fixes a distal end part of the leg of the user 3 to the pedal 12. The fixing portion 13 is formed of a band fixed to the pedal 12. The band is wrapped around the distal end part of the leg of the user 3 so that the distal end part of the leg of the user 3 is fixed to the pedal 12.

The exercise device 1 in the first embodiment includes the pair of pedals 12 and the pair of fixing portions 13, but the exercise device 1 in the first embodiment may have a configuration including one pedal 12 and one fixing portion 13.

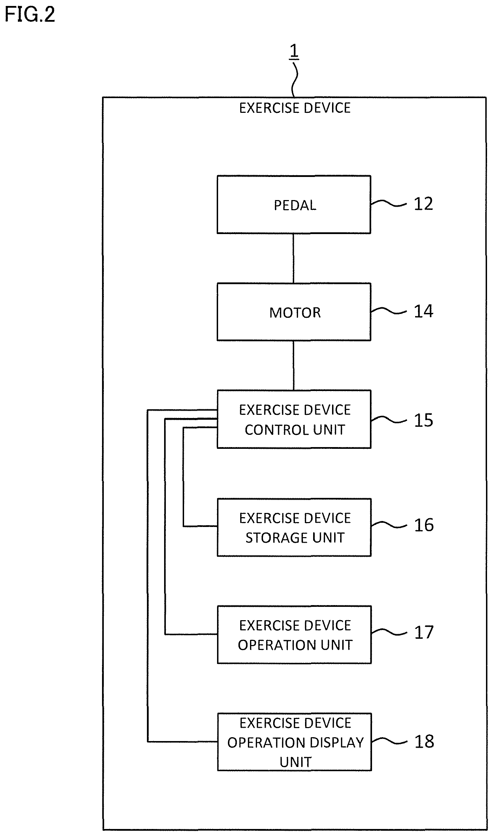

FIG. 2 is a block diagram for illustrating the configuration of the exercise device 1 of FIG. 1. The exercise device 1 further includes a motor 14, an exercise device control unit 15, an exercise device storage unit 16, an exercise device operation unit 17, and an exercise device operation display unit 18.

The pair of pedals 12 are connected to the motor 14. Through supply of currents to the motor 14, the motor 14 is driven. Through driving of the motor 14, the pair of pedals 12 are rotated. Accordingly, the pair of pedals 12 are rotated through supply of currents to the motor 14.

The exercise device control unit 15 executes a predetermined control program. The exercise device control unit 15 is formed of, for example, a central processing unit (CPU) or a micro processing unit (MPU). The exercise device control unit 15 controls the currents to be supplied to the motor 14. In this manner, the exercise device control unit 15 controls the rotation of the pair of pedals 12.

In the exercise device storage unit 16, various control programs and information to be used for various types of control processing are stored. The exercise device storage unit 16 is formed of, for example, a random access memory (RAM) or a read only memory (ROM). The exercise device storage unit 16 may be provided inside of the exercise device control unit 15.

Through operation of the exercise device operation unit 17, the number of revolutions of the pedals 12, the time period for which the pedals 12 are continuously rotated, and the like can be set for the exercise device control unit 15. As the number of revolutions of the pedals 12, 15 (rpm) to 30 (rpm) can be set.

The exercise device operation display unit 18 is formed of, for example, a liquid crystal panel. The exercise device operation display unit 18 displays contents and the like set through the exercise device operation unit 17.

The motor 14, the exercise device control unit 15, and the exercise device storage unit 16 are arranged inside of the exercise device main body 11. The exercise device operation unit 17 and the exercise device operation display unit 18 are arranged on the surface of the exercise device main body 11.

In the exercise device 1, the pedals 12 are rotated through the driving of the motor 14. The exercise device 1 may have a configuration in which switching is allowed between an automatic operation mode and a passive operation mode. The automatic operation mode is an operation mode in which the pedals 12 are rotated through the driving of the motor 14. The passive operation mode is an operation mode in which the pedals 12 are rotated through the pedaling exercise performed by the user 3.

As illustrated in FIG. 1, the electrical stimulation device 2 includes an electrical stimulation device main body 21, a plurality of electrodes 22, and a plurality of cables 23. The plurality of electrodes 22 are mounted to the leg of the user 3. The plurality of cables 23 connect the electrical stimulation device main body 21 to the respective electrodes 22.

Each of the electrodes 22 is a flat electrode. When the antagonistic muscle contracts and relaxes, the antagonistic muscle moves relative to the skin. Thus, when the antagonistic muscle contracts and relaxes, a motor point of the leg moves relative to the skin. The size of the surface of the electrode 22 to be brought into contact with the leg is set to a size that allows the electrode 22 to remain facing the motor point of the leg when the antagonistic muscle moves relative to the skin due to the contraction or the relaxing of the antagonistic muscle. With this configuration, even when the antagonistic muscle moves relative to the skin due to the contraction or the relaxing of the antagonistic muscle, electrical stimulation can be applied to the motor point of the leg from the electrode 22.

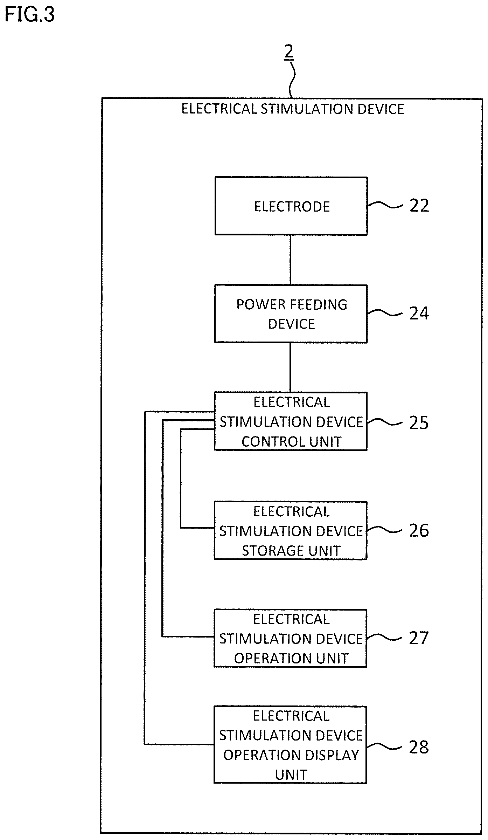

FIG. 3 is a block diagram for illustrating the configuration of the electrical stimulation device 2 of FIG. 1. The electrical stimulation device 2 further includes a power feeding device 24, an electrical stimulation device control unit 25, an electrical stimulation device storage unit 26, an electrical stimulation device operation unit 27, and an electrical stimulation device operation display unit 28.

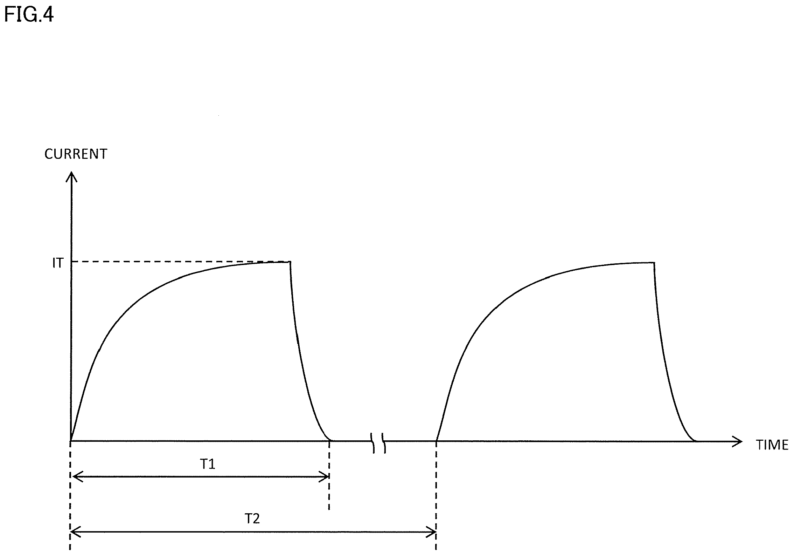

The power feeding device 24 supplies a current to each of the plurality of electrodes 22. FIG. 4 is a chart for showing a waveform of the current to be supplied to the electrode 22 of FIG. 1. The waveform of the current to be supplied to the electrode 22 is a pulse waveform. Further, the waveform of the current to be supplied to the electrode 22 is an exponentially gradually-increasing waveform. The current to be supplied to the electrode 22 gradually increases exponentially to reach a target value IT of the current as time advances. A pulse width T1 of the current to be supplied to the electrode 22 has a value set in advance. A period T2 of the current to be supplied to the electrode 22 has a value set in advance.

The plurality of electrodes 22 are connected in parallel to each other with respect to the power feeding device 24. In this manner, a current is supplied to each of the plurality of electrodes 22 from the power feeding device 24. Voltage values, current values, and frequencies to be applied to the plurality of respective electrodes 22 from the power feeding device 24, and application timings are the same among the plurality of electrodes 22. The voltage values, current values, and frequencies to be applied to the plurality of respective electrodes 22 from the power feeding device 24, and the application timings can be adjusted for each electrode 22.

As illustrated in FIG. 3, the electrical stimulation device control unit 25 executes a predetermined control program. The electrical stimulation device control unit 25 is formed of, similarly to the exercise device control unit 15, for example, a CPU or an MPU. The electrical stimulation device control unit 25 controls the current to be supplied to each of the plurality of electrodes 22 from the power feeding device 24. In this manner, the electrical stimulation device control unit 25 controls the electrical stimulation to be applied to the leg of the user 3.

In the electrical stimulation device storage unit 26, various control programs and information to be used for various types of control processing are stored. The electrical stimulation device storage unit 26 is formed of, similarly to the exercise device storage unit 16, for example, a RAM or a ROM. The electrical stimulation device storage unit 26 may be provided inside of the electrical stimulation device control unit 25.

Through operation of the electrical stimulation device operation unit 27, the current value to be supplied to the electrode 22, the frequency of the current to be supplied to the electrode 22, and the voltage value to be applied to the electrode 22 can be set for the electrical stimulation device control unit 25. Further, through operation of the electrical stimulation device operation unit 27, the pulse width of the current to be supplied to the electrode 22 and the number of times per second the current is to be supplied to the electrode 22 can be set for the electrical stimulation device control unit 25. Further, through operation of the electrical stimulation device operation unit 27, the number of times the electrical stimulation is to be given in one exercise therapy and a time period for which the electrical stimulation is to be given can be set for the electrical stimulation device control unit 25.

As the current value to be supplied to the electrode 22, 3 (mA) to 10 (mA) can be set. When the current value in a case in which the electrical stimulation is applied to the quadriceps femoris is equal to or smaller than 10 (mA), no stretching of the leg due to the electrical stimulation occurs. Further, when the current value in a case in which the electrical stimulation is applied to the hamstring is equal to or smaller than 10 (mA), no bending of the leg due to the electrical stimulation occurs. It is desired that the current value to be supplied to the electrode 22 be changed in accordance with the exercise target site to which the electrode 22 is mounted. When the electrode 22 is mounted to the thigh of the leg, 5 (mA) to 7 (mA) is desired. When the electrode 22 is mounted to the lower leg of the leg, 3 (mA) to 6 (mA) is desired.

When the electrical stimulation is applied to the quadriceps femoris so as to cause the stretching of the leg due to the electrical stimulation, a value exceeding 50 (mA) is required as the current value to be supplied to the electrode 22. When the electrical stimulation is applied to the hamstring so as to cause the bending of the leg due to the electrical stimulation, a value exceeding 50 (mA) is required as the current value to be supplied to the electrode 22.

As the frequency of the current to be supplied to the electrode 22, 5 (Hz) to 50 (Hz) can be set. As the frequency of the current to be supplied to the electrode 22, 20 (Hz) is desired. When the frequency of the current to be supplied to the electrode 22 is 20 (Hz), muscle fatigue of the exercise target site due to the electrical stimulation is less liable to occur. Thus, control of muscle contraction of the exercise target site caused by the electrical stimulation becomes easier. When the frequency of the current to be supplied to the electrode 22 is equal to or higher than 50 (Hz), muscle fatigue of the exercise target site due to the electrical stimulation is liable to occur. As a result, the muscle contraction of the exercise target site caused by the electrical stimulation cannot be sustained.

As the pulse width T1 of the current to be supplied to the electrode 22, 250 (ps) to 350 (µs) can be set. As the period T2 of the current to be supplied to the electrode 22, 20 (ms) to 200 (ms) can be set. As the frequency of the current to be supplied to the electrode 22, 50 (ms) is desired.

The electrical stimulation device operation display unit 28 is formed of, for example, a liquid crystal panel. The electrical stimulation device operation display unit 28 displays contents and the like set through the electrical stimulation device operation unit 27.

The power feeding device 24, the electrical stimulation device control unit 25, and the electrical stimulation device storage unit 26 are arranged inside of the electrical stimulation device main body 21. The electrical stimulation device operation unit 27 and the electrical stimulation device operation display unit 28 are arranged on the surface of the electrical stimulation device main body 21. The exercise therapy apparatus may have a configuration in which the exercise device main body 11 and the electrical stimulation device main body 21 are integrally formed. Further, the exercise therapy apparatus may have a configuration in which the exercise device operation unit 17 and the electrical stimulation device operation unit 27 are integrally formed. Further, the exercise therapy apparatus may have a configuration in which the exercise device operation display unit 18 and the electrical stimulation device operation display unit 28 are integrally formed.

In the exercise therapy apparatus according to the first embodiment, the electrical stimulation device 2 includes four electrodes 22 and four cables 23. Each of two electrodes 22 among the four electrodes 22 is referred to as "first electrode 221," and each of the remaining two electrodes 22 is referred to as "second electrode 222."

The two first electrodes 221 are mounted one by one to respective right and left legs of the user 3. As a method of mounting the first electrode 221 to the leg, there are given, for example, a method of attaching the first electrode 221 to the leg and a method of mounting the first electrode 221 to the leg by mounting the first electrode 221 to a supporter mounted to the leg. The first electrode 221 is mounted to a part corresponding to the quadriceps femoris in the leg of the user 3. Through supply of a current to the first electrode 221, a current is supplied to the quadriceps femoris of the leg of the user 3.

The two second electrodes 222 are mounted one by one to respective right and left legs of the user 3. As a method of mounting the second electrode 222 to the leg, there are given, for example, a method of attaching the second electrode 222 to the leg and a method of mounting the second electrode 222 to the leg by mounting the second electrode 222 to the supporter mounted to the leg. The second electrode 222 is attached to a part corresponding to the hamstring in the leg of the user 3. Through supply of a current to the second electrode 222, a current is supplied to the hamstring of the leg of the user 3.

The timing to supply the current to the electrode 22 is the timing unrelated to the position of the pedal 12. Accordingly, the electrical stimulation is applied to the quadriceps femoris and the hamstring regardless of the form of the leg. The form of the leg includes a leg bending form and a leg stretching form.

Not only when the leg stretches but also when the leg bends in the pedaling exercise, a current is supplied to the first electrode 221. A current having a value that does not cause stretching of the leg by the electrical stimulation is supplied to the first electrode 221. Accordingly, even when a current is supplied to the first electrode 221 in a case in which the leg bends in the pedaling exercise, the pedaling exercise is not hindered by the electrical stimulation.

Not only when the leg bends but also when the leg stretches in the pedaling exercise, a current is supplied to the second electrode 222. A current having a value that does not cause bending of the leg by the electrical stimulation is supplied to the second electrode 222. Accordingly, even when a current is supplied to the second electrode 222 in a case in which the leg stretches in the pedaling exercise, the pedaling exercise is not hindered by the electrical stimulation.

Next, the exercise therapy using the exercise therapy apparatus according to the first embodiment is described. First, the user 3 sits on the seat 4, fixes the distal end parts of the legs of the user 3 to the pedals 12, and mounts the first electrodes 221 and the second electrodes 222 to the legs of the user 3.

After that, the user 3 operates the exercise device 1 so as to rotate the pedals 12. In this manner, the legs of the user 3 start the pedaling exercise. When the pedals 12 are rotated, the user 3 is not required to move his or her legs by his or her own will. Accordingly, the pedaling exercise using the exercise device 1 is a completely passive exercise for the user 3.

After that, when a time period set in advance, for example, three minutes have elapsed from the start of the pedaling exercise using the exercise device 1, the user 3 operates the electrical stimulation device 2 so that currents are supplied to the first electrodes 221 and the second electrodes 222. In this manner, electrical stimulation is applied to the legs of the user 3. The current value to be supplied to the electrode 22 is a value that does not cause bending and stretching of the leg of the user 3 by the electrical stimulation. Accordingly, even when currents are supplied to the first electrodes 221 and the second electrodes 222, no strength for performing the pedaling exercise is generated in the legs of the user 3 performing the pedaling exercise.

The current value to be supplied to the electrode 22 is a value that does not cause bending and stretching of the leg of the user 3 by the electrical stimulation, and thus the pedaling exercise of the legs of the user 3 using the exercise device 1 is smoothly continued.

After that, when a time period set in advance, for example, 130 seconds have elapsed from the start of the electrical stimulation applied by the electrical stimulation device 2, the electrical stimulation applied by the electrical stimulation device 2 is ended. In synchronization with the end of the electrical stimulation, the user 3 operates the exercise device 1 so as to stop the rotation of the pedals 12. Thus, the exercise therapy using the exercise therapy apparatus according to the first embodiment is ended. When there occurs a situation in which it is difficult to continue the exercise such as a situation in which the user 3 feels bad, the user 3 can operate the electrical stimulation device 2 and the exercise device 1 without waiting for the elapse of the time period set in advance so as to stop the electrical stimulation and the rotation of the pedals 12.

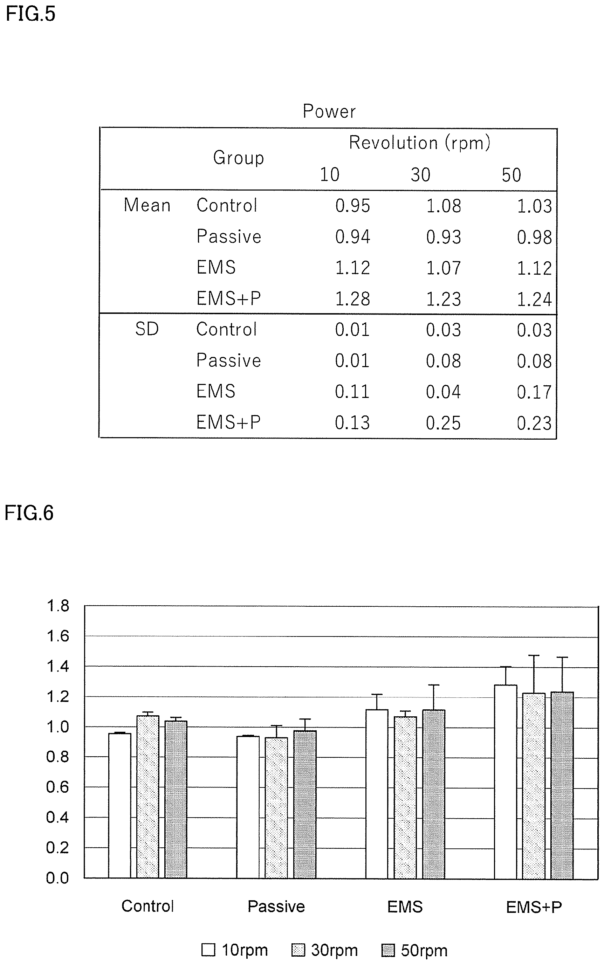

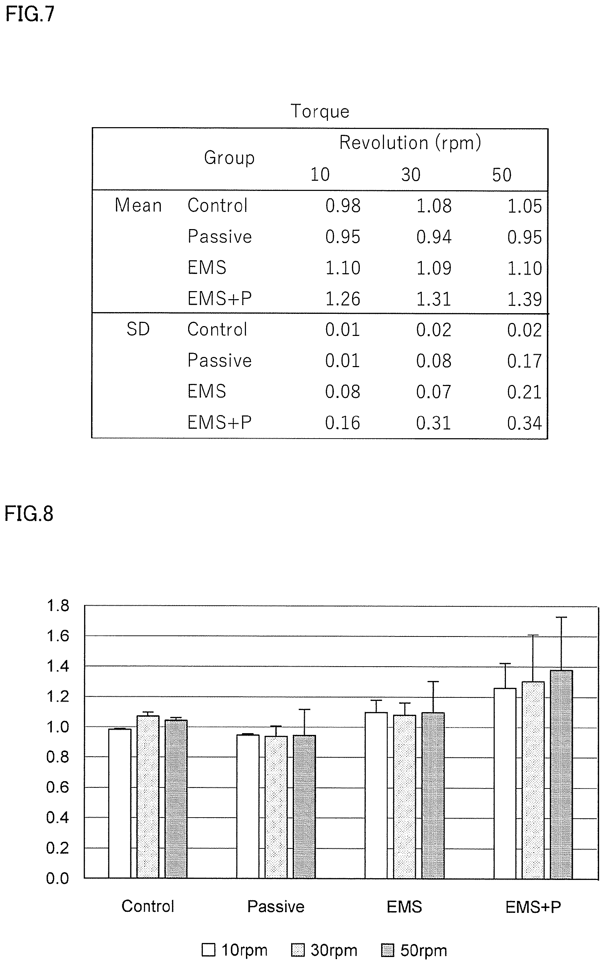

The inventors of this disclosure measured the muscle strengths of the user 3 before and after getting the exercise therapy using the exercise therapy apparatus according to the first embodiment. FIG. 5 is a table for showing a ratio of muscle strengths of the user 3 before and after getting the exercise therapy using the exercise therapy apparatus according to the first embodiment. FIG. 6 is a bar graph for showing the ratio of the muscle strengths of the user 3 of FIG. 5. FIG. 7 is a table for showing a ratio of muscle strengths of the user 3 before and after getting the exercise therapy using the exercise therapy apparatus according to the first embodiment. FIG. 8 is a bar graph for showing the ratio of the muscle strengths of the user 3 of FIG. 7.

In FIG. 5 to FIG. 8, "Control" indicates a group of people not performing training, and "Passive" indicates a group of people performing only a passive isokinetic pedaling exercise. Further, in FIG. 5 to FIG. 8, "EMS" indicates a group of people receiving only electrical stimulation at rest in a supine position posture, and "EMS+P" indicates a group of people performing a passive isokinetic pedaling exercise while receiving the electrical stimulation. Further, in FIG. 5 to FIG. 8, the value of the muscle strength is indicated through use of the ratio of the value of the muscle strength obtained after the exercise therapy is performed to the value of the muscle strength obtained before the exercise therapy is performed. Further, in FIG. 5 to FIG. 8, "Mean" indicates an average value, and "SD" indicates a standard deviation.

As shown in FIG. 5 to FIG. 8, an increase of the muscle strength in a case of performing the exercise therapy of causing the legs to mechanically exercise while applying electrical stimulation to the legs is larger than an increase of the muscle strength in a case of performing the exercise therapy of only causing the legs to mechanically exercise. Further, the increase of the muscle strength in the case of performing the exercise therapy of causing the legs to mechanically exercise while applying electrical stimulation to the legs is larger than an increase of the muscle strength in a case of performing the exercise therapy of only applying the electrical stimulation to the legs.

As described above, the exercise therapy apparatus according to the first embodiment includes the exercise device 1 for causing the leg of the user 3 to mechanically exercise, and the electrical stimulation device 2 for applying electrical stimulation to the quadriceps femoris and the hamstring included in the leg of the user 3. In the exercise therapy apparatus, the exercise device 1 causes the leg to mechanically exercise while the electrical stimulation device 2 applies electrical stimulation to each of the quadriceps femoris and the hamstring at the same time. With this configuration, a knee angle sensor is not required to measure the angle of the knee in the leg of the user 3, and the control device is not required to control supply of currents to the electrodes based on the angle of the knee. As a result, the configuration of the exercise therapy apparatus can be simplified.

Further, in the exercise therapy apparatus according to the first embodiment, the exercise device 1 causes the leg of the user 3 to mechanically exercise. In this manner, even when the leg of the user 3 cannot be freely moved due to paralysis, electrical stimulation can be applied to the leg of the user 3 while the leg of the user 3 is caused to mechanically exercise. As a result, even a user 3 who cannot freely move his or her leg due to paralysis can perform the exercise therapy using the exercise therapy apparatus.

Further, the exercise device 1 includes the pedal 12 which is rotatable, and the motor 14 for rotating the pedal 12. This exercise device 1 causes the leg of the user 3 to mechanically exercise through rotation of the pedal 12. With this configuration, the leg of the user 3 can be caused to perform the pedaling exercise mechanically.

Further, the electrical stimulation device includes the first electrode 221, the second electrode 222, and the power feeding device 24 for supplying each of the first electrode 221 and the second electrode 222 with a current having the same value at the same timing. The first electrode 221 is provided on the leg of the user 3 so as to correspond to the quadriceps femoris of the leg of the user 3. The second electrode 222 is provided on the leg of the user 3 so as to correspond to the hamstring of the leg of the user 3. With this configuration, each of the quadriceps femoris and the hamstring of the leg of the user 3 can be supplied with a current having the same value at the same timing.

Further, the value of the current to be supplied to the electrode from the power feeding device 24 is from 3 (mA) to 10 (mA). With this configuration, the electrical stimulation to be applied to the quadriceps femoris and the hamstring causes no bending and stretching exercise of the leg. In this manner, it is possible to suppress hindering of the pedaling exercise of the leg of the user 3 performed through use of the exercise device 1 due to the electrical stimulation applied by the electrical stimulation device 2.

Further, the frequency of the current to be supplied to each of the first electrode 221 and the second electrode 222 from the power feeding device 24 is 20 (Hz). With this configuration, muscle fatigue of the quadriceps femoris and the hamstring is less liable to occur. Thus, control of muscle contraction of the quadriceps femoris and the hamstring can be easily performed.

In the first embodiment, as the exercise target site of the body for which the exercise therapy apparatus is to be used, the leg of the user 3 has been described as an example. However, this disclosure is not limited thereto, and the exercise target site of the body for which the exercise therapy apparatus is to be used may be, for example, an arm of the user 3.

Further, in the first embodiment, the configuration of the exercise device 1 for causing the leg to perform the pedaling exercise mechanically has been described. However, this disclosure is not limited thereto, and it suffices that the exercise device 1 is configured to cause the leg to perform a bending and stretching exercise mechanically.

### Reference Signs List

1 exercise device, 2 electrical stimulation device, 3 user, 4 seat, 11 exercise device main body, 12 pedal, 13 fixing portion, 14 motor, 15 exercise device control unit, 16 exercise device storage unit, 17 exercise device operation unit, 18 exercise device operation display unit, 21 electrical stimulation device main body, 22 electrode, 23 cable, 24 power feeding device, 25 electrical stimulation device control unit, 26 electrical stimulation device storage unit, 27 electrical stimulation device operation unit, 28 electrical stimulation device operation display unit, 221 first electrode, 222 second electrode

## Claims

1. An exercise therapy apparatus, comprising:
an exercise device configured to cause an exercise target site of a body to mechanically exercise; and
an electrical stimulation device configured to apply electrical stimulation to a pair of antagonistic muscles included in the exercise target site regardless of a form of the exercise target site,
wherein the exercise device is configured to cause the exercise target site to mechanically exercise while the electrical stimulation device applies electrical stimulation to each of the pair of antagonistic muscles at the same time.

2. The exercise therapy apparatus according to claim 1,
wherein the exercise device includes:
a pedal which is rotatable; and
a motor configured to rotate the pedal, and
wherein the exercise device is configured to cause the exercise target site to mechanically exercise through rotation of the pedal.

3. The exercise therapy apparatus according to claim 1 or 2, wherein the electrical stimulation device includes:
a first electrode provided on the exercise target site so as to correspond to one of the pair of antagonistic muscles;
a second electrode provided on the exercise target site so as to correspond to the other of the pair of antagonistic muscles; and
a power feeding device configured to supply each of the first electrode and the second electrode with a current having the same value at the same timing.

4. The exercise therapy apparatus according to claim 3, wherein the value of the current to be supplied to each of the first electrode and the second electrode from the power feeding device is from 3 (mA) to 10 (mA).

5. The exercise therapy apparatus according to claim 3 or 4, wherein a frequency of the current to be supplied to each of the first electrode and the second electrode from the power feeding device is 20 (Hz).
